# EUROPÄISCHE PATENTANMELDUNG

(11) **EP 1 785 925 A2**
(43) Veröffentlichungstag der Anmeldung: **16.05.2007**
(21) Anmeldenummer: 06017939.7
(22) Anmeldetag: 29.08.2006
(51) Int. Cl.: G06Q 10/00

(54) **Verfahren und Architektur zur Anwendung elektronischer Formulare im medizinischen Bereich**

(30) Priorität: 14.11.2005 DE 102005054671
(71) Anmelder: Deutsche Telekom AG, 53113 Bonn (DE)
(72) Erfinder: Langer, Guido, 26127 Oldenburg (DE); Wulff, Wilhelm, 28215 Bremen (DE)

(57) **Zusammenfassung**

Die Erfindung betrifft ein Verfahren zur Anwendung von elektronischen Formularen im medizinischen Bereich. Mit dem vorgeschlagenen Verfahren und einer entsprechenden Architektur können Formulare zur Erfassung medizinischer Daten auf der Grundlage CDA-basierter Vorlagen dezentral nutzbar gemacht werden.

Dazu werden erfindungsgemäß projektbezogen erstellte Formularvorlagen auf dezentraler Ebene mittels geeigneter Werkzeuge auf ihre CDA-Konformität, überprüft und, sofern diese gegeben ist, als elektronische Vorlage, unter Verknüpfung mit technischen und medizinisch-fachlichen Regeln, in eine CDAgerechte Form überführt und einer zentralen Instanz übergeben. Hier werden die Formularvorlagen verwaltet und für eine Nutzung auf dezentraler Ebene bereitgestellt. Die Nutzung erfolgt mittels eines dezentral gehaltenen Client oder einer Drittanwendung. Bei einem Aufruf durch den Client werden die Formularvorlagen als speicherbare Formulare mit CDA-Struktur zur Erfassung medizinischer Daten visualisiert. Erfasste Daten können mittels des Client erneut zur Ansicht gebracht, bearbeitet, abgespeichert, ausgedruckt und/oder an andere Stelle übertragen werden.

## Beschreibung

Die Erfindung betrifft ein Verfahren zur Anwendung von elektronischen Formularen im medizinischen Bereich, wobei die Anwendung derartiger Formulare die Erzeugung und Bereitstellung entsprechender Formularvorlagen sowie deren Abruf für eine Nutzung von auf den Formularvorlagen beruhenden Formularen zur Erhebung beziehungsweise Erfassung medizinischer Informationen zum Zwecke ihres Austausches umfasst. Insbesondere bezieht sich die Erfindung auf ein Verfahren zur Erzeugung und Bereitstellung CDA-basierter (CDA = Clinical Document Architecture) Vorlagen und zur dezentralen Nutzbarmachung auf ihnen basierender Formulare für das Gesundheitswesen und angrenzende, mit medizinischen Informationen befasste Bereiche. Gegenstand der Erfindung ist weiterhin eine zur Durchführung des Verfahrens geeignete Architektur.

Getrieben von medizinischen und wirtschaftlichen Zwängen vollzieht sich auch im Gesundheitswesen bereits seit einiger Zeit eine Vereinheitlichung bestimmter Abläufe und eine Standardisierung der diesen zugrunde liegenden Sachverhalte und Dokumente. Allerdings sind gerade bei der zuletzt genannten Standardisierung von Dokumenten beziehungsweise Formularen auch auf der elektronischen Ebene noch viele Schwierigkeiten zu überwinden. So ist festzustellen, dass trotz der Standardisierungsbemühungen die Schnittstellen einzelner Anwendungen, wie Anwendungen für Praxiscomputersysteme einerseits und Krankenhausinformationssysteme andererseits sowie von sowohl im Praxisbereich als auch im Krankenhausbereich benötigten Abrechnungs- und

Auswertesystemen, sehr heterogen und/oder proprietär oder teilweise überhaupt nicht verfügbar sind. Auch heute werden daher noch viele Formulare auf Papierbasis erstellt und ausgetauscht. Dies ist jedoch im Hinblick auf den Zeit- und Kostenaufwand sowie die Fehleranfälligkeit unökonomisch. Dabei wird insbesondere die Auswertung und Aufbereitung der medizinischen Informationen zur Gewinnung strukturierter Daten, welche von proprietären Primärsystemen bereitgestellt werden, sehr erschwert.

Im Krankenhaus- beziehungsweise im klinischen Bereich sind indes durch die Einführung der so genannten Clinical Document Architecture (CDA) bedeutsame Fortschritte erreicht worden. Bei der CDA handelt es sich um einen XML-basierten Standard zur Strukturierung medizinischer Daten. Dieser ist seinerseits Bestandteil des umfassenderen HL7-Standards (Health Level 7), welcher im klinischen Umfeld für den Datenaustausch verwendet wird. Näheres hierzu ist beispielsweise unter www.sciphox.de offenbart.

Die CDA kann von vielen derzeit verwendeten Krankenhausinformationssystemen unmittelbar importiert werden. Die von dem Standard unterstützte Strukturierung der Daten ermöglicht in vorteilhafter Weise deren anonymisierte oder pseudoanonymisierte weitere Auswertung zur Verwendung in Studien oder Registern. Bei dem Import entsprechender Dokumente erfolgt deren Validierung darauf, ob es sich um ein gültiges CDA-Dokument handelt, im Allgemeinen über entsprechende XML-Validierungs-Schemata. Nachteilig ist es jedoch, dass dabei lediglich eine Prüfung der Dokumentstruktur, nicht aber des Inhalts erfolgt, so dass eine Validierung aus medizinischer Sicht nicht gegeben ist. Weiterhin ist es als nachteilig anzusehen, dass die entsprechenden Validierungsschemata bei lokalen Anwendungen, wie Praxis-Verwaltungssystemen (PVS) oder Krankenhausinformationssystemen (KIS) jeweils lokal eingespielt werden müssen. Dies bedeutet für den Betreiber der Systeme einen immensen Aufwand.

Abseits davon gibt es in Form des so genannten Connectathon ein Werkzeug zur Überprüfung auf ausschließlich medizinisch-fachlicher Ebene. Eine beide vorgenannten Prüfungsmechanismen vereinigende Lösung ist derzeit nicht bekannt. Auch ist festzustellen, dass sich die CDA im Praxisbetrieb niedergelassener Ärzte bisher kaum etabliert hat, trotz auch dort festzustellender Standardisierungsbestrebungen. Hier herrschen weiterhin proprietäre Lösungen beziehungsweise Lösungen vor, welche auf ein abgeschlossenes System (beispielsweise Praxis oder Praxisgemeinschaft) beschränkt sind.

Aufgabe der vorliegenden Erfindung ist es, eine Lösung bereitzustellen, durch welche Formulare zur Erfassung medizinischer Daten auf der Grundlage CDA-basierter Vorlagen dezentral nutzbar gemacht werden können. Hierzu sind ein Verfahren und eine zur Durchführung des Verfahrens geeignete Architektur anzugeben.

Die Aufgabe wird durch ein Verfahren mit den Merkmalen des Hauptanspruchs gelöst. Eine die Aufgabe lösende Architektur ist durch die Merkmale des ersten Sachanspruchs charakterisiert. Vorteilhafte Aus- beziehungsweise Weiterbildungen der Erfindung sind durch die jeweiligen Unteransprüche gegeben.

Durch das erfindungsgemäße Verfahren wird die Aufgabe gelöst, indem:
1. projektbezogen erstellte, elektronische Formularvorlagen auf einer dezentralen Ebene mittels dazu geeigneter Werkzeuge auf ihre Verfahrenskonformität, im Hinblick auf ihre CDA-Konformität überprüft werden
2. die Formatvorlagen, sofern ihre CDA-Konformität gegeben ist, unter Verknüpfung mit technischen und medizinisch-fachlichen Regeln in eine CDA-gerechte Form überführt werden
3. die in die CDA-gerechte Form überführten Formularvorlagen einer zentralen Instanz übergeben werden, wo sie verwaltet und für die Nutzung auf dezentraler Ebene bereitgestellt werden
4. die Nutzung der Formularvorlagen auf dezentraler Ebene mittels eines dezentral gehaltenen Client oder einer Drittanwendung erfolgt, wobei die Formularvorlagen bei einem Aufruf durch den Client als speicherbare Formulare mit CDA-Struktur zur Erfassung medizinischer Daten visualisiert werden und diese Formulare mittels des Client auch wiederholt sowie mit darin erfassten Daten erneut zur Ansicht gebracht, bearbeitet, abgespeichert, ausgedruckt und/oder an andere Stelle übertragen werden können.

Gemäß dem sich daraus in seiner Gesamtheit ergebenden Ablauf wird von einer Institution, welche in dem erfindungsgemäßen Verfahrensgang einzuspeisende Formularvorlagen in Umlauf bringt, zunächst ein Projekt definiert. Diesem Projekt werden eine oder mehrere entsprechende Formularvorlagen zugeordnet, wobei im Rahmen des Projekts das Layout der Formularvorlagen und Regeln für ihren Gebrauch formuliert werden. Dabei werden die dem Projekt zuzuordnenden Formularvorlagen entweder unmittelbar in elektronischer Form erzeugt oder zunächst in Papierform erstellt und danach durch einen Scannvorgang in die elektronische Form überführt. Die elektronischen Formularvorlagen werden dann mittels eines speziellen, dem Anbieter der Vorlagen zur Verfügung stehenden Vorlagengenerators auf ihre Konformität zum System überprüft. Hierbei erfolgt eine Überprüfung der Formularvorlagen unter technischen, durch die CDA und den ihr zugrunde liegenden XML-Standard bestimmten Gesichtspunkten. Ist die Konformität gegeben, werden die Formularvorlagen unter Berücksichtigung technischer, sich aus der CDA ergebender Regeln und der projektbezogenen medizinisch-fachlichen Regeln durch den Vorlagengenerator in eine CDA-gerechte Form überführt. Die CDA-gerechten Formularvorlagen werden schließlich in Form von Dateien der zentralen, vorzugsweise von einem Server in einem Netzwerk gehosteten Instanz übergeben, wo sie zentral für einen Abruf zur dezentralen Benutzung bereitgestellt werden. Dabei wirkt die zentrale Instanz quasi als Vorlagenverteiler und als Bindeglied zwischen dem Anbieter der Formularvorlagen und deren Nutzern, welches sicherstellt, dass alle Teilnehmer des Gesamtsystems auf identische Vorlagen zugreifen können. Die Kategorien dezentraler Nutzer beziehungsweise dezentraler Anbieter sowie zentrale Instanz sollen später noch anhand des Ausführungsbeispiels erläutert werden.

Nach den vorstehenden Darstellungen ist es ein Wesen der Erfindung, dass zwischen dem Anbieter der Formularvorlagen, bei dem es sich gegebenenfalls auch um den Verwender der mittels der auf den Formularvorlagen basierenden Formulare erhobenen Informationen handelt, und den Nutzern der Formularvorlagen, welche diese mit den zu erhebenden Informationen füllen, keine direkte Kommunikation erforderlich ist, aber dennoch, insbesondere auch unabhängig von der Beschaffenheit der lokal verwendeten Komponenten, für alle Nutzer die Nutzung system- beziehungsweise verfahrenskonformer Formularvorlagen zur Erhebung in sich konsistenter Daten ermöglicht wird. Die Nutzung der Formularvorlagen erfolgt dabei mittels eines speziellen Client oder durch Drittanwendungen, also gegebenenfalls auch durch proprietäre Lösungen, für welche eine entsprechende Schnittstelle in der Art eines API (Application Programming Interface) bereitgestellt wird, aber in jedem Falle auf dezentraler

Ebene.

Vorzugsweise können die Formularvorlagen durch ihren Anbieter dem Nutzer über die zentrale Instanz auch nutzerabhängig bereitgestellt werden. Hierbei werden in den Formularvorlagen Merkmale hinterlegt, welche den Kreis der zur Nutzung einer jeweiligen Formularvorlage vorgesehenen Nutzer spezifizieren oder die Nutzung der Formulare von einer dazu bestehenden Berechtigung abhängig machen. Dabei ist es denkbar, dass Formularvorlagen zum Beispiel unter medizinischen Gesichtspunkten fachrichtungsbezogen zur Verfügung gestellt werden, dass etwa ausschließlich für Internisten vorgesehene Formularvorlagen einem Orthopäden nicht zur Verfügung gestellt werden oder dass bestimmte Formularvorlagen nur dazu berechtigten Vertragsärzten von Versicherungen zugänglich gemacht werden.

Entsprechend einer vorteilhaften Weiterbildung des erfindungsgemäßen Verfahrens können im Zuge der Überführung der Formularvorlagen in die CDA-gerechte Form auch Workflows mit den Formularvorlagen verknüpft werden, welche von dem Vorlagenanbieter im Zusammenhang mit dem den Formularvorlagen zugrunde liegenden Projekt definiert werden. Diese Workflows werden der zentralen Instanz in Form von entsprechenden Steuerinformationen mit den Formularvorlagen übergeben und beim Aufruf der entsprechenden Formularvorlagen durch den Client des Nutzers mit übernommen. Die Visualisierung der auf entsprechenden Formularvorlagen basierenden Formulare durch den Client erfolgt dann, bezogen auf den mit der Formularvorlage verknüpften Workflow, kontextsensitiv. Die Verknüpfung einer Formularvorlage mit einem entsprechenden Workflow kann gemäß einer Ausbildungsform der Erfindung deren Verknüpfung mit anderen, in einem medizinischen Kontext zu ihr stehenden Formularvorlagen umfassen. Entsprechend werden bei einem Aufruf einer der miteinander verknüpften Formularvorlagen durch den Client das zugehörige Formular und, nach einer durch den Workflow festgelegten Reihenfolge, die weiteren, auf den verknüpften Formularvorlagen basierenden Formulare visualisiert.

Die Bereitstellung der Formularvorlagen umfasst gemäß einer vorteilhaften Ausbildung der Erfindung zudem eine Versionierung, die es ermöglicht, nicht nur aktuelle Vorlagen abzurufen, sondern im Bedarfsfall auch ältere Vorlagen informationen bereitzustellen. Entsprechend einer vorteilhaften Weiterbildung des Verfahrens ist es weiterhin vorgesehen, dass nicht nur die Formularvorlagen selbst durch Drittanwendungen, genutzt werden können, sondern auch der speziell zu ihrer Nutzung ausgebildete Client durch Drittbeziehungsweise Fremdanwendungen angesprochen werden kann. Dies erfolgt dabei über eine zusätzliche Schnittstelle des entsprechenden Client, über welche dieser parametrisiert, das heißt mit durch die Drittanwendung übergebenen Parametern, gestartet werden kann.

Eine die Aufgabe lösende und zur Durchführung des Verfahrens geeignete Architektur umfasst Komponenten zur Überführung elektronischer Formularvorlagen in eine CDA-gerechte Form, Komponenten zur Verwaltung und

Verteilung der CDA-gerechten Formularvorlagen sowie Komponenten zur Nutzung dieser Formularvorlagen für die Erfassung medizinischer Informationen. Erfindungsgemäß handelt es sich hierbei um dezentrale Komponenten mindestens eines Formularvorlagenanbieters zu Generierung CDA-gerechter Formularvorlagen, eine zentrale, durch entsprechende technische Komponenten abgebildete Instanz zur Verwaltung und Verteilung dieser Formularvorlagen und um wiederum dezentrale elektronische Komponenten eines oder mehrerer Nutzer für den Zugriff auf die zentrale Instanz und zur Nutzung der über die zentrale Instanz bereitgestellten Formularvorlagen. Dabei ist die vom Anbieter der Formularvorlage dezentral zu verwendende Komponente durch einen hard- und software-basierten Vorlagengenerator ausgebildet. Dieser beispielsweise durch einen PC mit Netzwerkanschluss und eine darauf ablaufende Software realisierte Vorlagengenerator umfasst Routinen, mittels welcher projektbezogen erstellte elektronische Formularvorlagen auf ihre CDA-Konformität überprüfbar und gegebenenfalls in eine CDA-gerechte Form überführbar sind. Weiterhin verfügt der Vorlagengenerator über Mittel zur Konnektierung der zentralen Instanz und für den Upload der CDA-Formularvorlagen auf entsprechende Komponenten der besagten zentralen Instanz. Bei der zentralen Instanz handelt es sich vorzugsweise um einen auf einem Server in einem Netzwerk (beispielsweise dem Internet) gehosteten Dienst. Dieser umfasst einen zentralen Transaktionsdienst zur Entgegennahme, Verwaltung und Bereitstellung der CDA-Formularvorlagen sowie ein Administrationsmodul. Die wiederum dezentral, bei dem oder den Nutzern gehaltenen Komponenten zur Verwendung der Formularvorlagen umfassen zumindest ein zur Kommunikation mit dem zentralen Dienstmodul und

Drittanwendungen ausgebildetes Schnittstellenmodul, einen lokalen Transaktionsdienst, ein Administrationsmodul sowie den eigentlichen, bereits mehrfach erwähnten CDA-Formular-Client, wobei die vorgenannten Komponenten, abgesehen von physischen beziehungsweise Hardwareeinheiten für einen Zugriff auf das zentrale Netz, im Wesentlichen durch entsprechende Softwaremodule realisiert sind. Die Zentrale Instanz wirkt als Bindeglied zwischen den Komponenten des dezentralen Anbieters und den dezentralen Komponenten der Nutzer der Formularvorlagen. Durch den zentralen Transaktionsdienst, welcher durch das zugehörige Administrationsmodul administrier- und konfigurierbar ist, werden die von dem (mindestens einem) Vorlagenanbieter empfangenen CDA-Formularvorlagen verwaltet und zur Abholung durch dezentrale Nutzer bereitgestellt. Dabei beinhaltet die Verwaltung gemäß vorteilhafter Ausbildungen des zugrunde liegenden Verfahrens gegebenenfalls auch die Bereitstellung älterer Versionen entsprechender Formularvorlagen.

Der korrespondierende lokale Transaktionsdienst, welcher durch entsprechende Hard- und/oder Softwaremittel bei einem dezentralen Nutzer realisiert ist, verwaltet, ähnlich dem zentralen Transaktionsdienst, die von diesem abgerufenen Formularvorlagen auf dezentraler Ebene. Zusätzlich ist er, im Zusammenhang mit der Visualisierung der auf den Formularvorlagen basierenden Formulare, für die Erstellung der den Formularen unterlegten eigentlichen CDA-Struktur und für deren Konsistenz verantwortlich. Über den lokalen, selbstverständlich ebenfalls wieder mittels des zugehörigen Administrationsmoduls administrierbaren Transaktionsdienst werden die Formularvorlagen für eine Nutzung mit dem CDA-Client auf lokaler Ebene bereitgestellt. Zudem ist durch diese zwischengeschaltete Verwaltung der Formularvorlagen auf dezentraler Ebene sichergestellt, dass ein Zugriff auf die Formularvorlagen auch im Falle eines System- oder Netzausfalls gegeben ist. Insoweit verfügt der lokale Transaktionsdienst über vergleichbare Mittel zur Datenhaltung wie der mit ihm korrespondierende zentrale Transaktionsdienst. Der CDA-Client, bei dem es sich vorzugsweise um eine Browserapplikation oder um einen Rich-Client handelt, visualisiert die auf den Formularvorlagen basierenden Formulare zur Erfassung der medizinischen Informationen in einer editierbaren Form. Über ihn ist erforderlichenfalls auch ein späterer erneuter Zugriff auf bereits mit Daten gefüllte Formulare möglich. Entsprechende Formulare können mittels des Client bearbeitet, ausgedruckt oder auch an andere Stelle übertragen werden.

Gemäß einer vorteilhaften Weiterbildung der Erfindung ist den dezentralen, beim Nutzer realisierten Komponenten eine Workflow-Engine beigeordnet. Die zentrale Instanz nimmt dabei entgegen und verwaltet Steuerinformationen, welche ihr durch den dezentralen Formularvorlagenanbieter im Zusammenhang mit Formularvorlagen übergeben werden, die mit entsprechenden Workflows verknüpft sind. Auf der Anwender- beziehungsweise Nutzerseite sorgt die Workflow-Engine dafür, dass die Formularvorlagen in der dem Workflow entsprechenden Reihenfolge und Verknüpfung visualisiert werden. Entsprechend einer ebenfalls vorgesehenen Weiterbildung der Erfindung kann den dezentralen Komponenten des Nutzers der Formularvorlagen außerdem ein weiteres auf den CDA-Client zugeschnittenes Schnittstellenmodul zugeordnet sein, mit welchem es möglich ist, den CDA-Client mit Fremdanwendungen zu nutzen. Selbstverständlich müssen entsprechende Fremdanwendungen über eine komplementäre Schnittstelle verfügen und geeignet sein, dem Client Parameter zu übergeben, durch welche die Kommunikation mit ihnen ermöglicht ist.

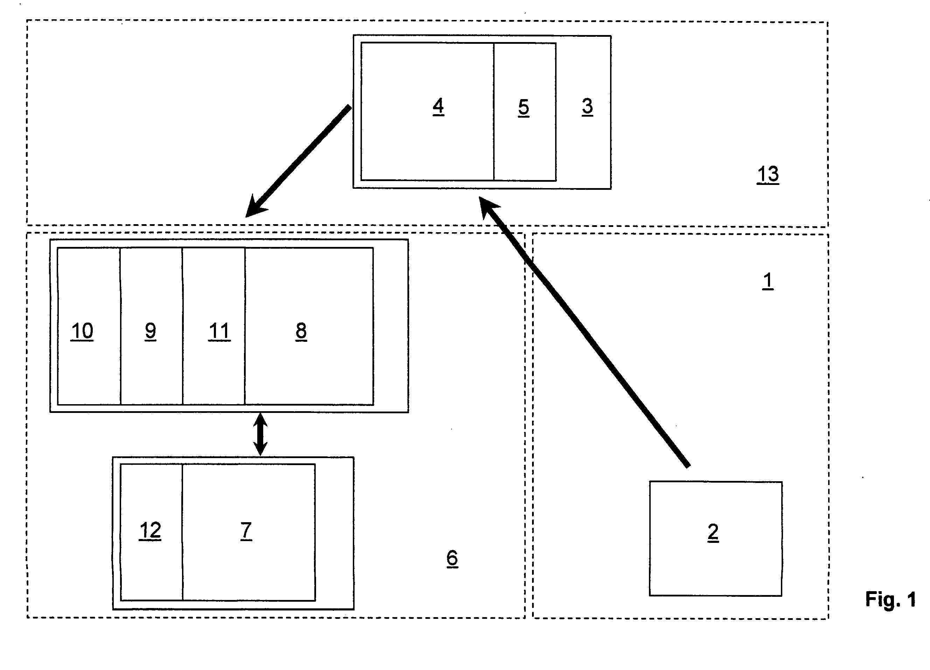

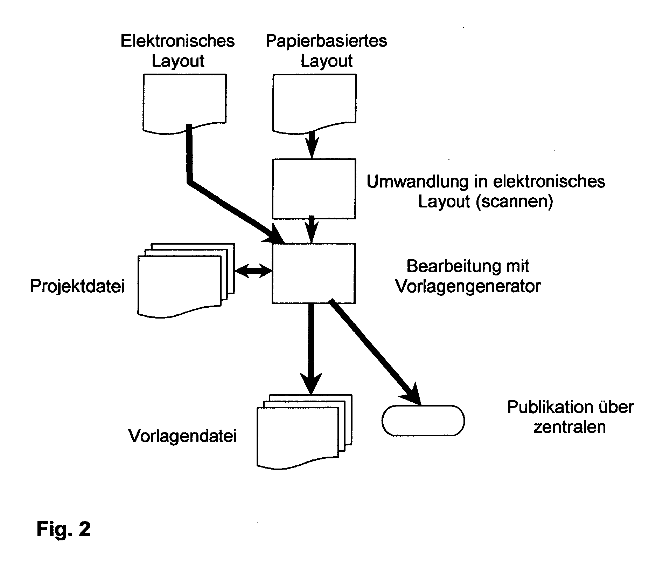

Einzelheiten der Erfindung sollen anhand der Fig. 1 und 2 nochmals erläutert werden.

Die Fig. 1 veranschaulicht die erfindungsgemäße Architektur in der Form eines groben Blockschaltbildes. Dargestellt sind der beim dezentralen Anbieter 1 der Formularvorlagen gehaltene Vorlagengenerator 2, die ebenfalls dezentral, bei einem oder mehreren Nutzern 6 der vom Anbieter 1 erstellten Formularvorlagen gehaltenen Komponenten und die zentrale, als Bindeglied zwischen den dezentralen Komponenten der Nutzer und des Anbieters fungierende Instanz 3 beziehungsweise das Dienstmodul. Bei dem dezentralen Anbieter 1 kann es sich beispielsweise um ein Krebsregister handeln, welches zur Erfassung der im Register zu hinterlegenden medizinischen Informationen spezielle Formularvorlagen in den Umlauf bringt. Die dazu projektbezogen erarbeiteten Formularvorlagen werden als elektronische Vorlagen in dem Formulargenerator 2 auf ihre CDA-Tauglichkeit geprüft und, wenn diese gegeben ist, in eine CDA-gerechte Form umgewandelt sowie schließlich an die zentrale Instanz 3, genauer gesagt an deren Transaktionsdienst 4 übergeben, welcher mit Hilfe eines Administrationsmoduls 5 administriert wird. Bei der zentralen Instanz 3 handelt es sich insoweit um ein Bindeglied, welches rein technischer Natur ist und beispielsweise in Form eines, in ein Netzwerk 13 einbezogenen, entsprechende Komponenten hostenden Servers mit zugehörigen Softwarekomponenten realisiert ist. Die Wirkungsweise der zentralen Instanz 3 als solches ist daher unabhängig von dem medizinischen Bezug der Erfindung. Bei dem dezentralen Nutzer 6 kann es sich um eine Arztpraxis oder um einen Praxisverbund handeln, dessen Praxen mit mehreren Computerworkstations ausgestattet sind, auf denen die für die Nutzung des Verfahrens erforderlichen Komponenten dezentral gehalten werden. Vorzugsweise werden die Formularvorlagen durch den zentralen Transaktionsdienst 4 der zentralen Instanz 3 nutzerabhängig verwaltet und bereitgestellt. Das meint beispielsweise, dass die Formularvorlagen fachrichtungsbezogen verwaltet werden. Hierdurch wird den Nutzern der Formularvorlagen das Auffinden der für ihre spezielle medizinische Fachrichtung gedachten Formularvorlagen erleichtert. So ist es denkbar, dass einem Orthopäden, welcher mittels des bei ihm installierten CDA-Formular-Client 7 über den lokalen Transaktionsdienst 8 auf den zentralen Transaktionsdienst 4 zugreift, nur solche Formulare zum Download angeboten werden, welche aus dem Blickwinkel der Orthopädie für ihn relevant sind. Gemäß dem Ausführungsbeispiel umfassen die dezentralen, beim Nutzer 6 gehaltenen Komponenten zudem eine Workflow-Engine 11 zur Nutzung von mit Workflows verknüpften Formularvorlagen. Die Komponenten des dezentralen Nutzers 6 kommunizieren mit der zentralen Instanz über das Schnittstellenmodul 10, über welches nicht nur dem speziellen Client 7, sondern auch auf der dezentralen Ebene gehaltenen Dritt- oder Fremdanwendungen der Zugriff auf den dezentralen Transaktionsdienst 8 gegeben ist. Der dezentrale Transaktionsdienst ist ebenfalls mittels eines ihm zugeordneten Administrationsmoduls 9 administrierbar. In dem dargestellten Beispiel ist außerdem dem CDA-Formularclient 7 ein weiteres Schnittstellenmodul 12 zugeordnet, über welches der Client 7 von Drittanwendungen durch einen parametrisierten Aufruf gestartet werden kann.

Der Prozess der Vorlagenerstellung und der im Rahmen des erfindungsgemäßen Verfahrens erfolgenden Überführung der erstellten Formulare in die CDA-gerechte Form mit anschließender Übergabe an die zentrale Instanz 3 ist durch das Ablaufschema in der Fig. 2 veranschaulicht. Danach werden die Vorlagen, wie mehrfach betont, immer projektbezogen, unmittelbar in elektronischer Form erstellt (linker Zweig) oder zunächst auf Papier dargestellt und durch Scannen in ein elektronisches Layout umgewandelt (rechter Zweig). Unabhängig von der Art ihrer Erstellung werden die letztlich in jedem Falle elektronischen Formulare mittels des Vorlagengenerators in eine CDA-gerechte Form überführt. Dabei werden Vorlagendateien und zugehörige Projektdateien erzeugt, welche auf lokaler Ebene gespeichert werden. Die Vorlagendateien beherbergen den eigentlichen inhaltlichen Kontext, während die zugehörigen Projektdateien zusätzliche Rohdaten, wie Hintergrundbilder und dergleichen, beinhalten. Unmittelbar nachfolgend oder zu einem späteren Zeitpunkt (daher die Möglichkeit der Speicherung der Vorlagen- und Projektdateien auf lokaler Ebene) werden die Formularvorlagen durch Übergabe an den zentralen Transaktionsdienst 4 publiziert, wobei diese in Form der Vorlagendateien an die zentrale Instanz 3 übergeben werden.

### Verwendete Bezugszeichen

- 1: dezentraler Anbieter
- 2: Vorlagengenerator
- 3: zentrale Instanz
- 4: zentraler Transaktionsdienst
- 5: Administrationsmodul
- 6: dezentraler Nutzer
- 7: Client
- 8: dezentraler Transaktionsdienst
- 9: Administrationsmodul
- 10: Schnittstellenmodul
- 11: Workflow-Engine
- 12: Schnittstellenmodul
- 13: Netzwerk beziehungsweise Netz

## Patentansprüche

1. Verfahren zur Anwendung elektronischer Formulare im medizinischen Bereich, nach welchem CDA-basierte Formularvorlagen erzeugt, bereitgestellt und für die Erhebung medizinischer Daten mittels Formularen auf der Grundlage der entsprechenden Vorlagen nutzbar gemacht werden, **dadurch gekennzeichnet, dass** projektbezogen erstellte Formularvorlagen auf dezentraler Ebene mittels dazu geeigneter Werkzeuge auf ihre Verfahrenskonformität, insbesondere ihre CDA-Konformität, überprüft und, sofern diese gegeben ist, als elektronische Vorlage, unter Verknüpfung mit technischen und medizinisch-fachlichen Regeln, in eine CDA-gerechte Form überführt und einer zentralen Instanz übergeben werden, wo sie verwaltet und für eine Nutzung auf dezentraler Ebene, mittels eines dezentral gehaltenen Client oder einer Drittanwendung, bereitgestellt werden, wobei die Formularvorlagen bei einem Aufruf durch den Client als speicherbare Formulare mit CDA-Struktur zur Erfassung medizinischer Daten visualisiert werden, welche mittels des Client auch wiederholt sowie mit darin erfassten Daten erneut zur Ansicht gebracht, bearbeitet, abgespeichert, ausgedruckt und/oder an andere Stelle übertragen werden können.

2. Verfahren nach Anspruch 1, **dadurch gekennzeichnet, dass** die Formularvorlagen nutzerabhängig bereitgestellt werden, wobei in den Formularvorlagen Merkmale hinterlegt werden, welche den Kreis der zur Nutzung einer jeweiligen Formularvorlage vorgesehenen und/oder berechtigten Nutzer spezifizieren.

3. Verfahren nach Anspruch 1 oder 2, **dadurch gekennzeichnet, dass** die Formularvorlagen im Zuge ihrer Überführung in die CDA-gerechte Form mit Workflows verknüpfbar sind, wobei bei der Übergabe einer mit einem Workflow verknüpften Formularvorlage an die zentrale Instanz Steuerinformationen mit übergeben werden, welche beim Aufruf der entsprechenden Formularvorlage durch den Client mit übernommen werden, so dass die Visualisierung auf dieser Formularvorlage basierender Formulare durch den Client, bezogen auf den mit der Formularvorlage verknüpften Workflow, kontextsensitiv erfolgt.

4. Verfahren nach Anspruch 3, **dadurch gekennzeichnet, dass** die Verknüpfung einer Formularvorlage mit einem Workflow deren Verknüpfung mit anderen, in einem medizinischen Kontext zu ihr stehenden Formularvorlagen umfasst, so dass bei einem Aufruf einer der verknüpften Formularvorlagen durch den Client das zugehörige Formular und entsprechend einer, durch den Workflow festgelegten Reihenfolge, die auf den verknüpften Formularvorlagen basierenden Formulare visualisiert werden.

5. Verfahren nach einem der Ansprüche 1 bis 4, **dadurch gekennzeichnet, dass** durch die zentrale Instanz gegebenenfalls Versionen einer jeweiligen Formularvorlagen verwaltet und zum Abruf bereitgestellt werden, so mittels des dezentral gehaltenen Client im Bedarfsfall auch Formularvorlagen mit älteren Vorlageninformationen abrufbar sind.

6. Verfahren nach einem der Ansprüche 1 bis 5, **dadurch gekennzeichnet, dass** der dezentrale Client zum Abruf von Formularvorlagen über Drittanwendungen nutzbar ist, welche den Client dazu unter Übergabe von Parametern an eine entsprechende Schnittstelle des Client starten.

7. Verfahren nach einem der Ansprüche 1 bis 6, **dadurch gekennzeichnet, dass** die projektbezogenen Formularvorlagen unmittelbar elektronisch oder auf Papier erstellt und durch einen Scann-Vorgang in die elektronische Form überführt werden.

8. Architektur zur Anwendung elektronischer Formulare im medizinischen Bereich, mit elektronischen Komponenten zur Überführung elektronischer Formularvorlagen in eine CDA-gerechte Form, Komponenten zur Verwaltung und Verteilung der CDA-gerechten Formularvorlagen sowie Komponenten zur Nutzung dieser Formularvorlagen für die Erfassung medizinischer Informationen, wobei die vorgenannten Komponenten hard- und/oder softwarebasiert sind, **dadurch gekennzeichnet, dass** zur Überführung der elektronischen Formularvorlagen in CDA-gerechte Formularvorlagen bei mindestens einem Anbieter (1) ein dezentral gehaltener Vorlagengenerator (2) ausgebildet ist und ein oder mehrere, von dem dezentralen Anbieter (1) sowie gegebenenfalls untereinander unabhängige Nutzer (6) über einen, bei ihnen dezentral gehaltenen Client (7) verfügen, durch welchen die von dem Vorlagengenerator (2) generierten CDA-Formularvorlagen als CDA-gerechte Formulare zur Erhebung medizinischer Daten visualisierbar sind, wobei als Bindeglied zwischen dem dezentralen Vorlagengenerator (2) und den dezentralen Clients (7) ein zentrales Dienstmodul (3) mit einem zentralen Transaktionsdienst (4) zur Verwaltung und Verteilung von Formularvorlagen und mit einem Administrationsmodul (5) zum Administrieren des zentralen Transaktionsdienstes (4) ausgebildet ist, auf welches der Vorlagegenerator (2) zur Übergabe der von ihm generierten Formularvorlagen und die Clients (7) zum Abruf von Formularvorlagen Zugriff haben und wobei den Clients (7) auf der dezentralen Ebene ein lokaler Transaktionsdienst (8) zur dezentralen Verwaltung vom zentralen Dienstmodul abgerufener Formularvorlagen, ein Administrationsmodul (9) zur Administration des lokalen Transaktionsdienstes (8) sowie ein Schnittstellenmodul (10) zur Kommunikation mit dem zentralen Dienstmodul (3) und mit Drittanwendungen zugeordnet ist.

9. Architektur nach Anspruch 8, **dadurch gekennzeichnet, dass** den Clients auf der dezentralen Ebene zusätzlich eine Workflow-Engine (11) zugeordnet ist, mittels welcher durch den Vorlagengenerator (2) mit einem Workflow verknüpfte Formularvorlagen, bezogen auf den entsprechenden Workflow, durch den Client (7) kontextsensitiv visualisierbar sind.

10. Architektur nach Anspruch 8 oder 9, **dadurch gekennzeichnet, dass** der Client (7) über ein weiteres Schnittstellenmodul (12) für Drittanwendungen verfügt, vermittels welchem der Client (7) über einen parametrisierten Aufruf durch die Drittanwendungen nutzbar ist.
